Europäisches Patentamt

European Patent Office

Office européen des brevets

(19)

(11) Veröffentlichungsnummer : **0 065 123**
**B1**

(12) # EUROPÄISCHE PATENTSCHRIFT

(45) Veröffentlichungstag der Patentschrift :
25.07.84

(21) Anmeldenummer : 82103471.7

(22) Anmeldetag : 24.04.82

(51) Int. Cl.³ : **C 07 H 15/20**, C 07 H 17/06,
A 61 K 31/70

(54) Schwer lösliche Salze von Aminoglykosidantibiotika.

(30) Priorität : 13.05.81 DE 3118856
25.02.82 DE 3206725

(43) Veröffentlichungstag der Anmeldung :
24.11.82 Patentblatt 82/47

(45) Bekanntmachung des Hinweises auf die Patenterteilung : 25.07.84 Patentblatt 84/30

(84) Benannte Vertragsstaaten :
AT BE CH DE FR GB IT LI NL SE

(56) Entgegenhaltungen :
US-A- 3 091 572
CHEMICAL ABSTRACTS, Band 87, Nr. 21, 21. November 1977, Seite 4, Nr. 161337b, Columbus Ohio (USA);

(73) Patentinhaber : **Merck Patent Gesellschaft mit beschränkter Haftung**
**Frankfurter Strasse 250**
**D-6100 Darmstadt (DE)**

(72) Erfinder : **Wahlig, Helmut, Dr.**
**Roemheidweg 16**
**D-6100 Darmstadt (DE)**
Erfinder : **Dingeldein, Elvira, Dr.**
**Am Spitzenpfad 9**
**D-6072 Dreieich (DE)**
Erfinder : **Kirchlechner, Richard, Dr.**
**Im Ritterbruch 15**
**D-6146 Alsbach-Hähnlein 2 (DE)**
Erfinder : **Orth, Dieter, Dr.**
**Carl-Ulrichstrasse 35**
**D-6100 Darmstadt (DE)**
Erfinder : **Rogalski, Werner, Dr.**
**Rosengartenstrasse 15**
**D-6146 Alsbach (DE)**

**Beschreibung**

Die Erfindung betrifft neue schwer lösliche Salze von Aminoglykosidantibiotika.

Aminoglykosidantibiotika wie Gentamycin oder Tobramycin werden üblicherweise in Form ihrer Sulfate verwendet, die in Wasser leicht löslich sind. Die Antibiotika werden schnell aus ihnen freigesetzt und verteilen sich im Körper. Dieses Verhalten ist in manchen Fällen von Nachteil, wenn nämlich eine lokal begrenzte Infektion bekämpft werden soll, z. B. ein infizierter Knochen. In diesen Fällen sind schwerer lösliche Salze erwünscht, aus denen das Antibiotikum langsamer freigesetzt wird und die daher eine gewisse Depotwirkung entfalten können.

Einige schwer lösliche Salze von Aminoglykosidantibiotika sind bekannt. So sind z. B. in der US-PS 3 091 572 verschiedene schwer lösliche Salze des Gentamycins erwähnt (z. B. Salze mit Fettsäuren, die 8 oder mehr C-Atome enthalten, wie Laurin-, Stearin-, Palmitin- oder Ölsäure, Aralkansäuren wie Phenylbuttersäure, Arylcarbonsäuren wie Naphthalin-1-carbonsäure, Schwefel- und Sulfonsäuren wie Laurylschwefelsäure, Dodecylbenzolsulfonsäure).

Es hat sich gezeigt, daß diese Salze bei ihrer Anwendung gewisse Nachteile aufweisen. So zeigen sie eine wachsartige, deutlich hydrophobe Beschaffenheit, die ihre galenische Verarbeitung erschwert.

Der Erfindung lag die Aufgabe zugrunde, neue Salze von Antibiobiotika aufzufinden, die schwer löslich sind und die die nachteiligen Eigenschaften der bekannten Antibiotika-Salze nicht oder nur in geringerem Ausmaß aufweisen. Diese Aufgabe wurde durch die Bereitstellung der neuen Salze gelöst.

Es wurde gefunden, daß eine langsamere Freisetzung der Antibiotika erzielt werden kann, wenn man an Stelle der genannte Sulfate oder anderer leichtlöslicher Salze die schwer löslichen Flavanoidphosphate, insbesondere die Hesperidinphosphate der Aminoglykosidantibiotika einsetzt.

Gegenstand der Erfindung sind dementsprechend die Flavanoidphosphate, insbesondere die Hesperidinphosphate von Aminoglykosidantibiotika.

Als anionische Komponenten der erfindungsgemäßen Salze eignen sich saure Phosphorsäureester von Hydroxyflavanoiden, z. B. von Hydroxy-flavanen, -flavenen, -flavanonen, -flavonen oder -flavyliumsalzen. Die Flavanon- und Flavonderivate sind bevorzugt.

Dabei können die Hydroxyflavanoide eine oder mehrere, z. B. 1,2,3,4,5,6 oder 7, vorzugsweise 1,2,3 oder 4, Hydroxygruppen enthalten, die vorzugsweise phenolischer Art sind, aber auch alkoholischer Art sein können. Sie stehen in der Regel in 3-, 5-, 6-, 7-, 3'- und/oder 4'-Stellung der Flavansystems, können aber auch in 4-, 8-, 2'-, 5'- oder 6'-Stellung stehen. Bevorzugt sind die 3'- und die 5-Stellung. Von den Hydroxygruppen können eine oder mehrere mit Phosphorsäure verestert sein. So können z. B. der 3'- und der 5-Monophosphorsäureester sowie der 3',5-Diphosphorsäureester des Hesperidins als Salzbildungskomponenten verwendet werden. Der Ausdruck « Herperidinphosphorsäure » soll sich im folgenden auf den 3',5-Diphosphorsäureester beziehen, der Ausdruck « Hesperidinphosphate » auf die davon abgeleiteten Salze.

Außer den phosphorylierten und freinen OH-Gruppen können die Flavanoidphosphorsäuren weitere Substituenten tragen, z. B. veretherte OH-Gruppen wie Alkoxygruppen mit vorzugsweise 1-4 C-Atomen, vor allem Methoxygruppen (in der Regel nicht mehr als drei, vorzugsweise eine, bevorzugt in der 4'-Stellung, aber auch in der 3-, 3'-, 5-, 6- und/oder 7-Stellung), sowie insbesondere glykosidierte OH-Gruppen. Diese können mit Mono-, Di-, Tri- oder Tetrasacchariden glykosidiert sein. Als Glykosidkomponenten bevorzugt sind Monosaccharide wie D-Glucose ferner D-Galactose, D-Glucuronsäure, D-Galacturonsäure, D-Xylose, D-Apiose, L-Rhamnose und L-Arabinose, sowie Disaccharide wie Rhamnosylglucosen, besonders bevorzugt Rutinose und Neohesperidose, ferner z. B. Rungiose, Robinobiose, Sophorose, Gentiobiose, Apiobiose, Vicianose, Sambubiose, Primverose oder Latyrose. Glykosidierte OH-Gruppen stehen vorzugsweise in 7- und/oder 3-Stellung ; in der Regel sind höchstens 2, vorzugsweise eine glykosidierte OH-Gruppe im Molekül der Flavanoidphosphorsäure vorhanden. Weitere mögliche Substituenten (in der Regel nicht mehr als 3, vorzugsweise nur einer) sind z. B. Alkyl mit z. B. 1-4 C-Atomen, vorzugsweise Methyl, Halogen, vorzugsweise F oder Cl, Hydroxyalkoxy mit z. B. 1-4 C-Atomen, vorzugsweise 2-Hydroxyethoxy.

Als Flavanoidphosphate eignen sich im einzelnen z. B. saure Phosphorsäureester von Hydroxyflavanen wie 6-Hydroxy-4'-methoxyflavan, 6-Hydroxy-3,4'-dimethoxyflavan, 6-Hydroxy-4'-methoxy-3-methylflavan, Catechin ((+)-3,3',4',5,7-Pentahydroxyflavan), Leucocianidol (3,3',4,4',5,7-Hexahydroxyflavan), und deren Glykosiden wie 2,3,3',4,4',5,7-Heptahydroxyflavanglucosid ; Hydroxyflavanonen wie Liquiritigenin (4',7-Dihydroxyflavanon), Pinocembrin (Dihydrochrysin, 5,7-Dihydroxyflavanon), Naringenin (4',5,7-Trihydroxyflavanon), Eriodictyol (3',4',5,7-Tetrahydroxyflavanon), Dihydroquercetin (Taxifolin, 3,3',4',5,7-Pentahydroxyflavanon), 6-Hydroxy-4'-methoxy-flavanon, Sakuranetin (4'5-Dihydroxy-7-methoxy-flavanon), Isosakuranetin (5,7-Dihydroxy-4'-methoxy-flavanon), Hesperetin (3',5,7-Trihydroxy-4'-methoxyflavanon), Silibinin (2-[trans-2-(4-Hydroxy-3-methoxyphenyl)-3-hydroxymethyl-1,4-benzodioxan-6-yl]-3,5,7-trihydroxychroman-4-on), und deren Glykosiden wie Pinocembrin-7-rutinosid, Sarothanosid (Pinocembrin-7-neohesperidosid), Salipurposid (Naringenin-5-glucosid), Prunin (Naringenin-7-glucosid), Narirutin (Naringenin-7-rutinosid), Naringin (Naringenin-7-neohesperidosid), Eriodictin (Eriodictyol-7-rhamnosid), Eriocitrin (Eriodictyol-7-rutinosid), Eriodictyol-7-neohesperidosid, Didymin (Isosakuranetin-7-rutinosid), Poncirin (Isosakuranetin-7-neohesperidosid), Persicosid (Hesperitin-glucosid), Hesperidin

(Hesperetin-7-rutinosid), Neohesperidin (Hesperetin-7-neohesperidosid) ; Hydroxyflavonen wie Chrysin (5,7-Dihydroxyflavon), Primetin (5,8-Dihydroxyflavon), Galangin (3,5,7-Trihydroxyflavon), Baicalein (5,6,7-Trihydroxyflavon), Apigenin (4',5,7-Trihydroxyflavon), Datiscetin (2',3,5,7-Tetrahydroxyflavon), Lotoflavin (2',4',5,7-Tetrahydroxyflavon), Kämpferol (3,4',5,7-Tetrahydroxyflavon), Fisetin (3,3',4',7-Tetrahydroxyflavon), Luteolin (3',4',5,7-Tetrahydroxyflavon), Scutellarein (4',5,6,7-Tetrahydroxyflavon), Morin (2',4,4',5,7-Pentahydroxyflavon), Robinetin (3,3',4',5',7-Pentahydroxyflavon), Quercetin (3,3',4',5,7-Pentahydroxyflavon), Tectochrysin (5-Hydroxy-7-methoxyflavon), Genkwanin (4',5-Dihydroxy-7-methoxyflavon), Acacetin (5,7-Dihydroxy-4'-methoxyflavon), Diosmetin (3',5,7-Trihydroxy-4'-methoxyflavon), Chrysoeriol (4',5,7-Trihydroxy-3'-methoxyflavon), Rhamnetin (3,3',4',5-Tetrahydroxy-7-methoxyflavon), Isorhamnetin (3,4',5,7-Tetrahydroxy-3'-methoxyflavon), Chlorflavonin (3'-Chlor-2',5-dihydroxy-3,7,8-trimethoxyflavon), Eupatorin (3',5-Dihydroxy-4',6,7-trimethoxyflavon) und deren Glykosiden wie Chrysin-7-rutinosid, Chrysin-7-neohesperidosid, Apiin (Apigenin-7-apiosylglucosid), Rhoifolin (Apigenin-7-neohesperidosid), Isorhoifolin (Apigenin-7-rutinosid), Nicotiflorin (Kämpferol-3-rutinosid), Lespedin (Kämpferol-3,7-dirhamnosid), Robinin (Kämpferol-3-robinosid-7-rhamnosid), Scolymosid (Lonicerin, Luteolin-7-rutinosid), Veronicastrosid (Luteolin-7-neohesperidosid), Quercitrin (Quercetin-3-rhamnosid), Isoquercitrin (Quercetin-3-glucosid), Hyperosid (Quercetin-3-galactosid), Rutosid (Rutin, Quercetin-3-rutinosid), 6-Hydroxymethylrutosid, Monoxerutin [7-(2-Hydroxyethyl)-rutosid], Ethoxazorutosid [4'-0-(2-Morpholinoethyl)-rutosid], Troxerutin [3',4',7-Tris-(2-hydroxyethyl)-rutosid], Acaciin (Linarin, Acacetin-7-rutinosid), Fortunellin (Acacetin-7-neohesperidosid), Diosmin (Diosmetin-7-rutinosid), Neodiosmin (Diosmetin-7-neohesperidosid), Narcissin (Isorhamnetin-3-rutinosid) ; Hydroxyflavyliumsalzen wie Cyanidin, und deren Glykosiden wie Keracyanin (Cyanidin-3-rutinosid).

Als Aminoglykosidantibiotika kommen insbesondere diejenigen in Betracht, die eine Desoxystreptamin-Einheit enthalten. Im einzelnen sind besonders bevorzugt Amikacin, Dibekacin, Gentamycin, Neomycine, Paromomycin, Sagamycin, Sisomicin, Streptomycin und Tobramycin, weiterhein sind z. B. bevorzugt Allomycin, Amicetin, Apramycin, Bekanamycin, Betamicin, Butirosin, Destomycin, Evernimomycine, Ezomycine, Flambamycin, Fortimycin A und B, Framycetin, Hikizimycin, Homomycin, Hybrimycin, Hygromycin, Kanamycine, Kasugamycin, Lividomycin, Minosaminomycin, Myomycine, Netilmicin, Parvulomycin, Puromycin A, Ribostamycin, Rimocidin, Ristomycin, Rlstosamin, Seldomycine, Sorbistin, Spectinomycin, Streptothricin, Tunicamycin und Verdamycin sowie deren Epimeren und Derivate, soweit sie basisch sind.

Da einige dieser Antibiotika, z. B. Gentamycin, bekanntlich keine einheitlichen Substanzen sind, sondern Gemische (Gentamycin z. B. ein Gemisch der Verbindungen Gentamycin C 1, Gentamycin C 2 und Gentamycin C 1a) darstellen, sind auch die Flavanoidphosphate in einzelnen Fällen keine einheitlichen Substanzen, sondern Gemische. Da außerdem viele der genannten Antibiotika, z. B. alle Gentamycine, mehrere basische Stickstoffatome enthalten und da andererseits Flavanoidphosphorsäuren wie Hesperidinphosphorsäure mehrbasige Säuren sind, ist es außerdem möglich, daß sich saure, neutrale und/oder basische Salze bilden. Alle diese möglichen Salze und ihre Gemische untereinander sind in der Definition « Flavanoidphosphate von Aminoglykosidantibiotika » eingeschlossen.

Bevorzugt sind die neutralen Salze und diese enthaltende Gemische ; im Fall der Gentamycinhesperidinphosphate ist z. B. besonders bevorzugt das Salz (Gemisch) aus 2 Mol Gentamycin und 5 Mol Hesperidinphosphorsäure. (« Neutral » bedeutet hier, daß auf jeden Phosphorsäurerest eine basische Aminogruppe entfällt).

Gegenstand der Erfindung ist auch ein Verfahren zur Herstellung von Flavanoidphosphaten von Aminoglykosidantibiotika, dadurch gekennzeichnet, daß man ein wasserlösliches Salz eines Aminoglykosidantibiotikums mit einem Flavanoidphosphat oder einem seiner wasserlöslichen Salze umsetzt.

Diese Herstellung erfolgt in an sich bekannter Weise, etwa durch Zusammengeben einer wässerigen Lösung des wasserlöslichen Salzes des Antibiotikums (z. B. Gentamycinsulfat) mit einer wässerigen Lösung des Flavanoidphosphats oder eines seiner wasserlöslichen Salze (z. B. des Dinatriumsalzes), zweckmäßig unter Rühren bei Raumtemperatur. Zur Verbesserung der Löslichkeit kann auch ein organisches Lösungsmittel zugesetzt werden, z. B. ein Alkohol wie Ethanol. Die gebildeten Flavanoidphosphate sind in Wasser schwer löslich und können durch Abfiltrieren, Waschen mit Wasser und Trocknen erhalten werden.

Gegenstand der Erfindung ist ferner die Verwendung der genannten Flavanoidphosphate zur Herstellung pharmazeutischer Zubereitungen, insbesondere auf nicht-chemischem Wege. Hierbei können sie zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens ein Flavanoidphosphat eines Aminoglykosidantibiotikums.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen

0 065 123

Anwendung Suppositorien, zur parenteralen Anwendung Lösungen, Suspensionen, Emulsionen oder Implantate, für die topische Anwendung Salben, Cremes oder Puder. Implantate, beispielsweise auf der Basis von Silikonkautschuk, Tricalciumphosphat oder Kollagen, die sich z. B. zur Behandlung des infizierten Knochens eignen, sind von besonderer Bedeutung. Die neuen Verbindungen können auch lyophilisiert und die erhaltenen Lyophilisate z. B. zur Herstellung von Injektionspräparaten verwendet werden. Die angegebene Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. leicht lösliche Salze der gleichen oder anderer Antibiotika, um zusätzlich zu dem Depot-Effekt, der durch die Flavanoidphosphate bewirkt wird, eine systemische Wirkung zu erzielen.

Insbesondere ist Gegenstand der Erfindung ein neues Fibrin-Antibiotikum-Gel, das mindestens ein Flavanoidphosphat eines Aminoglykosidantibiotikums enthält.

Fibrin-Antibiotikum-Gele, die als Antibiotikum Tobramycin, Gentamycin und/oder eines ihrer physiologisch unbedenklichen Salze enthalten, sind aus der Internationalen Patentanmeldung WO 81/00516 bekannt. Als physiologisch unbedenkliche Salze der beiden Antibiotika sind dort spezifisch nur die Sulfate genannt. Diese bekannten Fibrin-Antibiotikum-Gele, die Tobramycinsulfat oder Gentamycinsulfat enthalten, zeigen jedoch bei der praktischen Anwendung, z. B. bei der Behandlung des infizierten Knochens, den Nachteil, daß die Antibiotika zu schnell aus ihnen freigesetzt werden. Die Antibiotika verteilen sich im Körper und werden zum Teil ausgeschieden ; sie können dann an der eigentlichen Infektionsstelle nicht mehr in dem gewünschten Maße wirksam werden. Das neue Fibrin-Antibiotikum-Gel zeigt diese nachteiligen Eigenschaften der bekannten Gele nicht oder nur in geringerem Ausmaß.

Zur Herstellung der Fibrin-Antibiotikum-Gele können die Gentamycinsalze in der so erhaltenen Form oder aber in feinverteilter, z. B. mikronisierter Form eingesetzt werden.

Die Fibrin-Antibiotikum-Gele können in an sich bekannter Weise hergestellt werden, zweckmäßig durch Mischen einer Fibrinogenlösung, einer Thrombinlösung und des neuen Flavanoidphosphats eines Aminoglykosidantibiotikums. Das Fibrin wird dabei ausgefällt. Die Thrombinlösung enthält zweckmäßig zusätzlich Aprotinin und/oder ist mit Calcium-Ionen angereichert, z. B. in Form von $CaCl_2$. Außer den Flavanoidphosphaten werden alle Bestandteile des Gels zweckmäßig in Form üblicher Handelspräparate verwendet. Es ist möglich, das Gel erst am Ort der Wahl, z. B. unmittelbar in der Knochenhöhle durch Zusatz der Thrombinlösung zu der Fibrinogenlösung zu bilden, wobei das Salz des Antibiotikums vorher entweder der Thrombinlösung oder der Fibrinogenlösung zugesetzt wird. Bevorzugt wird das Gel aber durch extrakorporale Mischung der Bestandteile hergestellt. In beiden Fällen kann man den Gerinnungsvorgang des Fibrins zeitlich steuern, indem man die Konzentration des Thrombins verändert.

Das Fibrinogen kann z. B. als humanes Fibrinogen in Form eines handelsüblichen Kryopräzipitats verwendet werden, das etwa 90 mg/ml thrombinfällbares Protein enthält, oder als Lyophilisat, z. B. aus menschlichem Blut von gepooltem Spenderplasma gewonnen. Das Fibrin-Antibiotikum-Gel enthält zweckmäßig etwa 2 bis etwa 10, vorzugsweise etwa 3 bis 6, Gewichtsprozent Fibrin.

Die Thrombinlösung wird zweckmäßig hergestellt, indem man Thrombin (z. B. in Form eines Pulvers) in einer wässerigen Calciumchlorid-Lösung auflöst. Diese kann z. B. 1 000 bis 10 000 KIE (Kallikrein-Inaktivator-Einheiten), vorzugsweise etwa 3 000 KIE Aprotinin pro ml enthalten. Die Konzentration an Calciumchlorid beträgt vorzugsweise etwa 20 bis 60, insbesondere etwa 40, mMol/l. Die Konzentration des Thrombins liegt vorzugsweise zwischen etwa 10 und etwa 500 NIH-Einheiten pro ml. Man verwendet zur Herstellung des Gels zweckmäßig etwa die gleichen Volumina Fibrinogenlösung und Thrombinlösung.

Das Salz des Antibiotikums wird zweckmäßig in einer auf das Körpergewicht bezogenen Menge eingesetzt, wobei die Tageshöchstdosis beachtet werden sollte. Die Konzentration des Antibiotikums in dem Fibrin-Antibiotikum-Gel liegt vorzugsweise zwischen etwa 0,5 und etwa 10, insbesondere zwischen 1 und 5 Gewichtsprozent, bezogen auf die Base Des Aminoglykosidantibiotikums.

Die Gerinnungszeit des Gels hängt von der Thrombin-Konzentration ab. So kann man die plastische Verformbarkeit des entstehenden Gerinnsels für die Dauer von 1/2 bis Minute aufrechterhalten, wenn man eine Thrombin-Konzentration von etwa 150 NIH-Einheiten pro ml verwendet. Die Fließeigenschaften des Gels werden durch niedrigere Thrombinkonzentration (10-15 NIH-Einheiten/ml) wesentlich länger aufrechterhalten (etwa bis zu 3 Minuten). Die Fibringerinnung wird dadurch verlangsamt, die Reißfestigkeit des Polymerisats eher erhöht.

Außer den erfindungsgemäß verwendbaren Salzen können in der Gelen zusätzlich noch andere physiologisch unbedenkliche Gentamycinsalze, z. B. das Sulfat, oder Gentamycin-Base enthalten sein, ferner auch andere Antibiotika, wie Tobramycin, Neomycin, Streptomycin, Penicilline, Bacitracin, Clindamycin und/oder deren physiologisch unbedenklichen Salze. Die Gele können auch weitere Wirkstoffe enthalten.

Das Fibrin-Antibiotikum-Gel bei primärer Spongiosaplastik beherrscht nicht nur den Infekt, sondern verbessert auch die osteogenetische Potenz des biologischen Implantates.

Es ist natürlich möglich, auch infektgefährdete Knochen, z. B. nach offenen Frakturen, zur Infektionsverhütung mit dem Fibrin-Antibiotikum-Gel zu behandeln. Dabei wird durch die Speziellen Gentamycinsalze ein besonders hoher lokaler Wirkstoff-Spiegel erzielt.

4

Die verzögerte Freisetzung des Antibiotikums aus den erfindungsgemäßen Fibrin-Antibiotikum-Gelen kann im Vergleich zu der Freisetzung aus mit Gentamycinsulfat erhaltenen Gelen in an sich bekannter Weise nachgewiesen werden, wobei man das freigesetzte Gentamycin vorzugsweise mikrobiologisch bestimmt. Diese Bestimmung kann in vitro erfolgen, z. B. durch Elution in wässeriger Pufferlösung oder tierischem oder menschlichem Serum. Es kann auch nach Implantierung des Gels in vivo oder nach erfolgter Knochen-Operation die Ausscheidungsgeschwindigkeit im Urin oder die zeitliche Veränderung der Konzentration im Serum oder in Geweben in der gleichen Weise bestimmt werden. In-vivo-Versuche können an beliebigen Versuchstieren, z. B. Ratten, Kaninchen oder Hunden, oder am Menschen vorgenommen werden.

Gegenstand der Erfindung ist ferner die Verwendung der genannten Flavanoidphosphate bei der Bekämpfung von Krankheiten, insbesondere von bakteriellen Infektionen, sowie ihre Verwendung bei der therapeutischen Behandlung des menschlichen oder tierischen Körpers.

Dabei werden die erfindungsgemäßen Substanzen vorzugsweise in Dosierungen zwischen etwa 5 und 1 000 mg, insbesondere zwischen 10 und 500 mg pro Dosierungseinheit (bezogen auf Antibiotikum-Wirkstoff) verabreicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die lokale Applikation ist bevorzugt.

In den nachfolgenden Beispielen sind die Temperaturen in °C angegeben.

### Beispiel 1

Eine Lösung von 7,07 g (10 mMol) Gentamycinsulfat in 200 ml Wasser wird bei 20° unter Rühren mit einer Lösung von 20,4 g (25 mMol) Hesperidin-5,3',-diphosphorsäureester-dinatriumsalz in 600 ml Wasser versetzt.

Man rührt noch eine Stunde, saugt das erhaltene Gentamycin-hesperidinphosphat (Gentamycin. 2,5 Hesperidinphosphat) ab, wäscht mit Wasser nach und trocknet über KOH. F. 227-229 ° (Zersetzung) ; IR-Spektrum (in KBr) : 3 410, 2 950, 1 637, 1 572, 1 510, 1 440 cm$^{-1}$.

### Beispiele 2 bis 8

Analog Beispiel 1 erhält man aus den berechneten Mengen der Sulfate der entsprechenden Antibiotika und Hesperidin-5,3'-diphosphorsäureester-dinatriumsalz :

2. Neomycin-hesperidinphosphat (= Neomycin. 3 Hesperidinphosphat), F. 228-230° (Zersetzung).

3. Paromomycin-hesperidinphosphat (= Paromomycin. 2,5 Hesperidinphosphat), F. 219-222° (Zersetzung).

4. Sisomycin-hesperidinphosphat (= Sisomycin. 2,5 Hesperidinphosphat), F. 220-221° (Zersetzung).

5. Amikacin-hesperidinphosphat (= Amikacin. 2 Hesperidinphosphat), F. 226-229° (Zersetzung).

6. Tobramycin-hesperidinphosphat (= Tobramycin. 2,5 Hesperidinphosphat), F. 228° (Zersetzung).

7. Dibekacin-hesperidinphosphat (= Dibekacin. 2,5 Hesperidinphosphat), F. 230° (Zersetzung).

8. Streptomycin-hesperidinphosphat (= Streptomycin. 3 Hesperidinphosphat), F. 212-213° (Zersetzung).

### Beispiel 9

Eine Lösung von 7,07 g g Gentamycinsulfat in 200 ml Wasser wird bei 20° unter Rühren zu einer Lösung von 17,5 g (50 mMol) 6-Hydroxy-4'-methoxy-flavanon-6-phosphorsäureester in 150 ml Ethanol und 1 600 ml Wasser gegeben. Man rührt noch eine Stunde, saugt das erhaltene Gentamycinsalz des 6-Hydroxy-4'-methoxy-flavanon-6-phosphorsäure-esters ab, wäscht mit Wasser nach und trocknet über KOH. F. 210-215° (Sintern bei 190°).

Die nachstehenden Beispiele betreffen pharmazeutische Zubereitungen, die Hesperidinphosphate von Aminoglykosidantibiotika enthalten :

### Beispiel A : Kapseln

Man füllt 10 kg Neomycin-hesperidinphosphat in üblicher Weise in Hartgelatinekapseln, so daß jede Kapsel Wirkstoff entsprechend 165 mg Neomycin-Base enthält.

### Beispiel B : Ampullen

Man mikronisiert 1 kg Gentamycin-hesperidinphosphat fein, suspendiert in 30 l Sesamöl und füllt in Ampullen ab, die unter sterilen Bedingungen verschlossen werden. Jede Ampulle enthält Wirkstoff entsprechend 10 (40, 80, 120) mg Gentamycin-Base.

Beispiel C : Implantate

Man mischt 1,54 g mikronisiertes Gentamycin-hesperidin-phosphat (entsprechend 0,2 g Gentamycin) mit 8,5 g Silikonkautschuk-Monomeren (Medical Grade Silastic 382, Dow Corning), gibt 2 Tropfen Polymerisationskatalysator hinzu, mischt erneut und formt zu runden Scheiben von 20 mm Durchmesser und 1 mm Dicke. Jede Scheibe enthält 6 mg Gentamycin-Base.

Beispiel D : Fibrin-Antibiotikum-Gel

Man löst 4 NIH-Einheiten Thrombin (Handelspräparat) in 1 ml Aprotinin-Calciumchlorid-Lösung (Handelspräparat ; 3 000 KIE/ml Aprotinin in 40 mMol/l CaCl$_2$) erwärmt die Lösung auf 37°, gibt eine Menge Gentamycin-hesperidin-phosphat, die 20 mg Gentamycin-Base entspricht, hinzu und mischt mit der gleichen Menge vorher auf 37° erwärmten « Fibrinkleber » (Handelspräparat ; hergestellt durch Kältepräzipitation aus humanem Spenderplasma ; bei − 18° oder kälter gelagert ; 1 ml der Lösung enthält durchschnittlich 90 mg thrombinfällbares Protein, Gesamteiweißgehalt der Lösung etwa 10 Gewichtsprozent ; etwa 20-30 Minuten vor der geplanten Verwendung aufgetaut). Man läßt des Gemisch in Zylindern aus rostfreiem Staht (Innendurchmesser 6 mm, Höhe 10 mm) erstarren (1 ml für 3 Zylinder). Die gebildeten Gel-Zylinder werden anschließend aus den Formen ausgestoßen.

**Ansprüche**

1. Flavanoidphosphate von Aminoglykosidantibiotika.
2. Hesperidinphosphate von Aminoglykosidantibiotika.
3. Gentamycin-hesperidinphosphat.
4. a) Neomycin-hesperidinphosphat.
   b) Paromomycin-hesperidinphosphat.
   c) Sisomycin-hesperidinphosphat.
   d) Amikacin-hesperidinphosphat.
   e) Tobramycin-hesperidinphosphat.
   f) Dibekacin-hesperidinphosphat.
   g) Streptomycin-hesperidinphosphat.
5. Verfahren zur Herstellung von Flavanoidphosphaten von Aminoglykosidantibiotika, dadurch gekennzeichnet, daß man ein wasserlösliches Salz eines Aminoglykosidantibiotikums mit einem Flavanoidphosphat oder einem seiner wasserlöslichen Salze umsetzt.
6. Verfahren zur Herstellung pharmazeutischer Zubereitungen, dadurch gekennzeichnet, daß man ein Flavanoidphosphat eines Aminoglykosidantibiotikums zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.
7. Pharmazeutische Zubereitung, gekennzeichnet durch einen Gehalt an mindestens einem Flavanoidphosphat eines Aminoglykosidantibiotikums.
8. Fibrin-Antibiotikum-Gel mit einem Gehalt an mindestens einem Flavanoidphosphat eines Aminoglykosidantibiotikums.
9. Flavanoidphosphate von Aminoglykosidantibiotika zur Bekämpfung von Krankheiten.

**Claims**

1. Flavonoid phosphates of aminoglycoside antibiotics.
2. Hesperidin-phosphates of aminoglycoside antibiotics.
3. Gentamycin hesperidin-phosphate.
4. a) Neomycin hesperidin-phosphate.
   b) Paromomycin hesperidin-phosphate.
   c) Sisomycin hesperidin-phosphate.
   d) Amikacin hesperidin-phosphate.
   e) Tobramycin hesperidin-phosphate.
   f) Dibekacin hesperidin-phosphate.
   g) Streptomycin hesperidin-phosphate.
5. Process for the preparation of flavonoid phosphates of aminoglycoside antibiotics, characterised in that a water-soluble salt of an aminoglycoside antibiotic is reacted with a flavonoid phosphate or one of its water-soluble salts.
6. Process for the preparation of pharmaceutical formulations, characterised in that a flavonoid phosphate of an aminoglycoside antibiotic is brought into a suitable dosage form together with at least one solid, liquid or semi-liquid excipient or auxiliary, if appropriate in combination with one or more other active compound(s).

7. Pharmaceutical formulation, characterised in that it contains at least one flavonoid phosphate of an aminoglycoside antibiotic.

8. Fibrin antibiotic gel containing at least one flavonoid phosphate of an aminoglycoside antibiotic.

9. Flavonoid phosphates of aminoglycoside antibiotics for combating illnesses.

**Revendications**

1. Flavanoïde-phosphates d'antibiotiques du type aminoglucoside.

2. Hespéridine-phosphates d'antibiotiques du type aminoglucoside.

3. L'hespéridine-phosphate de gentamycine.

4. a) L'hespéridine-phosphate de néomycine.
   b) L'hespéridine-phosphate de paromomycine.
   c) L'hespéridine-phosphate de sisomycine.
   d) L'hespéridine-phosphate d'amikacine.
   e) L'hespéridine-phosphate de tobramycine.
   f) L'hespéridine-phosphate de dibékacine.
   g) L'hespéridine-phosphate de streptomycine.

5. Procédé de préparation de flavanoïde-phosphates d'antibiotiques du type aminoglucoside, caractérisé en ce que l'on fait réagir un sel hydrosoluble d'un antibiotique du type aminoglucoside avec un flavanoïde-phosphate ou l'un de ses sels solubles dans l'eau.

6. Procédé de préparation de compositions pharmaceutiques, caractérisé en ce que l'on met sous une forme de dosage appropriée un flavanoïde-phosphate d'un antibiotique du type aminoglucoside avec au moins un véhicule ou produit auxiliaire solide, liquide ou semi-liquide et le cas échéant en combinaison avec une ou plusieurs autres substances actives.

7. Composition pharmaceutique, caractérisée en ce qu'elle contient au moins un flavanoïde-phosphate d'un antibiotique du type aminoglucoside.

8. Gel fibrine-antibiotique contenant au moins un flavanoïde-phosphate d'un antibiotique du type aminoglucoside.

9. Les flavanoïdes-phosphates d'antibiotiques du type aminoglucosides pour la lutte contre les maladies.